Europäisches Patentamt

European Patent Office

Office européen des brevets

(19)

(11) Numéro de publication: **0 123 585**
**B1**

(12) ## FASCICULE DE BREVET EUROPEEN

(45) Date de publication du fascicule du brevet: **31.08.88**

(51) Int. Cl.⁴: **A 61 L 15/03,** A 61 K 9/70

(21) Numéro de dépôt: **84400584.3**

(22) Date de dépôt: **22.03.84**

(54) **Nouvelle forme transdermale de médicaments.**

(30) Priorité: **24.03.83 FR 8304839**

(43) Date de publication de la demande:
**31.10.84 Bulletin 84/44**

(45) Mention de la délivrance du brevet:
**31.08.88 Bulletin 88/35**

(84) Etats contractants désignés:
**AT BE CH DE FR GB IT LI LU NL SE**

(56) Documents cités:
**EP-A-0 033 615**
**DE-A-3 231 400**
**FR-A-2 421 610**
**GB-A-2 095 108**
**US-A-3 996 934**

**CHEMICAL ABSTRACTS, vol. 96, no. 22, mai 1982, page 420, no. 187302x, Columbus, Ohio, US**

**Le dossier contient des informations techniques présentées postérieurement au dépôt de la demande et ne figurant pas dans le présent fascicule.**

(73) Titulaire: **RHONE-POULENC SANTE**
**Les Miroirs 18 Avenue d'Alsace**
**F-92400 Courbevoie Cédex (FR)**

(72) Inventeur: **Haggiage, Johnny**
**72 rue du Fort St. Irénée**
**F-69005 Lyon (FR)**
Inventeur: **Pusineri, Christian**
**33, rue des Fleurs**
**F-69360 Serezin du Rhône (FR)**

(74) Mandataire: **Pilard, Jacques et al**
**RHONE-POULENC RECHERCHES Service**
**Brevets Pharma 25, Quai Paul Doumer**
**F-92408 Courbevoie Cedex (FR)**

Courier Press, Leamington Spa, England.

# 0 123 585

**Description**

La présente invention concerne une nouvelle forme transdermale de médicaments.

Plus particulièrement, l'invention concerne une nouvelle forme galénique de médicaments choisis parmi les produits anti-angoreux (dinitrate d'isosorbide, mononitrate-5 d'isosorbide, trinitrine), antiémétiques (métopimazine, dimenhydrinate), anticholinergiques (scopolamine), anti-inflammatoires non stéroïdiens (kétoprofène), anti-inflammatoires stéroïdiens (prednisolone, dexaméthazone), anti-hypertenseurs (clonidine), béta-bloquants (acébutolol), vasodilatateurs (nicergoline), neuroleptiques (phénothiazines), tranquillisants (benzodiazépines, suriclone, suproclone), bronchodilatateurs (phénylpropanolamine), anti-spasmodiques (bromure de butylhyoscine) ou les hormones (estradiol).

Ces médicaments, actifs à des doses relativement faibles, nécessitent, pour présenter un maximum d'efficacité, d'être libérés progressivement dans l'organisme. Généralement ces médicaments sont utilisés sous forme de comprimés, de tablettes administrables par voie orale ou sublinguale ou de suppositoires. Utilisés sous cette forme les médicaments ont une durée d'action relativement courte, qui, pour certains d'entre eux, peut être inférieure à 1 heure. Un traitement continu nécessite la prise de produit à des intervalles de temps bien précis sans pour autant obtenir une action régulière. Il est particulièrement important de pouvoir disposer de formes galéniques qui assurent une libération continue et contrôlée du principe actif dans l'organisme de façon a avoir un effet aussi constant que possible pendant une période déterminée pouvant atteindre plusieurs heures ou même plusieurs jours.

A cet effet, il a été proposé des compositions agissant par absorption percutanée dans lesquelles les doses administrées peuvent être facilement contrôlées et qui libèrent de façon régulière le principe actif dans l'organisme. De telles formes galéniques facilitent l'administration du principe actif. Elles permettent un accès direct à la circulation systémique sans passage par le tractus gastro-intestinal et elles évitent l'effet de premier passage.

Divers systèmes ont été proposés pour la réalisation de compositions transdermales.

Le brevet américain US 3 996 934 concerne des compositions transdermiques dans lesquelles des microcapsules contenant le principe actif sont dispersées dans une matrice polymérique, le contrôle de la libération du principe actif pouvant être réalisé par adjonction d'une membrane microporeuse.

La demande de brevet allemand DE 3 214 900 concerne des compositions transdermiques constituées d'un polymère acrylique contenant le principe actif et d'un agent favorisant la diffusion du principe actif.

La demande de brevet européen EP 33 615 concerne des compositions transdermiques dans lesquelles un réservoir contenant le principe actif est recouvert d'une membrane permettant le contrôle de la libération.

La demande de brevet français FR 2 421 610 concerne des compositions administrables par voie buccale dans lesquelles la substance active est dispersée dans un homopolymère d'acide acrylique ou de vinylpyrrolidone ou d'un copolymère avec un acrylate.

La demande de brevet anglais GB 2 095 108 concerne des compositions transdermiques dans lesquelles le principe actif est dispersé dans un copolymère acrylique ayant une température de transition vitreuse comprise entre −70 et 10°C.

Selon le brevet américain US 3 797 494, la régulation du relargage est assurée par une membrane microporeuse. Le système est constitué d'une partie réservoir contenant le produit actif, d'une membrane micropore et d'un adhésif pour fixer le système sur la peau.

Il peut être aussi utilisé, comme décrit par exemple dans la demande de brevet européen 13606, un système ne comportant pas de membrane microporeuse, la peau du patient faisant office de membrane régulatrice de débit. Le principe actif est contenu dans un système à base d'alcool polyvinylique et de gel hydrosoluble à base de polyvinylpyrrolidone.

D'autres préparations sont décrites dans lesquelles sont utilisés des réticulats de silicones ou de polymères élastomériques comme, par exemple, dans les brevets français 2 497 457 ou américain 4 336 243.

Ces différents systèmes peuvent présenter des difficultés technologiques de mise en oeuvre ou procurent des résultats qui sont fonction de la sensibilité du patient, la peau faisant office de membrane régulatrice.

Il a maintenant été trouvé, et c'est ce qui fait l'objet de la présente invention, que les membranes microporeuses pouvaient être avantageusement remplacées par des membranes homogènes dont l'hydrophilie variable permet de contrôler la cinétique de libération du principe actif.

Les nouvelles formes transdermales selon l'invention se différencient des sytèmes connus par la nature des constituants et par la possibilité de réguler les cinétiques de libération du principe actif selon un processus qui ne fait pas appel à l'utilisation d'une membrane microporeuse.

Dans les nouvelles formes transdermales, le principe actif est dispersé sous forme cristallisée et/ou, de préférence, solubilisé dans une matrice élastomère homogène avec un agent amphiphile non ionique. En faisant varier le caractère hydrophile du mélange, il est possible de déterminer et de contrôler la vitesse de relargage du principe actif.

Pour la réalisation des formes transdermales selon l'invention il est particulièrement avantageux d'utiliser des polymères thermo-réversibles, non réticulés, filmogènes, insolubles dans l'eau mais éventuellement miscillables dont il est possible de faire varier le caractère hydrophile. Les films obtenus à

2

partir de ces polymères gonflent en présence d'eau (sous forme de liquide ou de vapeur) et le taux de gonflement peut être ajusté par la balance hydrophile-lipophile de la matrice, de l'additif et du produit actif.

A cet effet, conviennent plus spécialement des copolymères polyuréthane-polyoxyéthylèneglycol (POEG)-polyoxypropylèneglycol (POPG). Il peut être avantageux d'utiliser des polyuréthanes à base polyéther ou polyester de polytétraméthylèneglycols qui sont des produits commercialisés en particulier sous les noms de Pellethane (N.D. Upjohn), d'Estane (N.D. Goodrich).

D'une manière générale, le caractère hydrophile (ou hydrophobe) d'un copolymère peut être déterminé par le taux de gonflement en atmosphère humide de la manière suivante:

Un échantillon préalablement pesé est placé en atmosphère humide saturée pendant au moins une nuit à une température voisine de 20°C. Le poids de léchantillon gonflé permet de calculer le taux d'hydration qui est directement lié à l'hydrophilie du polymère.

Par exemple, dans le cas de·copolymères polyuréthane-POEG-POPG, le caractère hydrophile sera fonction du rapport POEG/POPG. Dans ce cas, le copolymère est hydrophile lorsque le rapport POEG/POPG est supérieur à 1 et il est hydrophobe lorsque le rapport POEG/POPG est inférieur à 1, la caractère hydrophile ou hydrophobe étant plus ou moins important selon la valeur exacte du rapport.

La dispersion du principe actif dans la matrice polymérique est assurée au moyen d'émulsifiants non ioniques, compatibles avec la matrice polymérique et avec le principe actif, lesdits émulsifiants étant généralement ceux utilisés en cosmétologie et en pharmacie comme ingrédients pour des crèmes, des laits ou des pommades ou comme agents gélifiants ou dispersants ou comme excipients pour suppositoires. Ces émulsifiants non ioniques, à base de polymères glycoliques ou glycéroliques, sont en général des condensats de polyoxydes d'éthylène avec des acides gras ou des alcools gras, des esters de sorbitane, des triglycérides à fonction hydroxyles libres, des monoglycérides polypropylèneglycol ou des esters de polyglycérol. Parmi ces émulsifiants non ioniques peuvent être en particulier utilisés des produits fabriqués et commercialisés par exemple le palmitostéarate de polyglycérol (Plurol stéarique, N.D. Gattefosse), l'isostéarate de polyglycérol (Plurol isostéarique, N.D. Gattefosse), le monopalmitate de saccharose (Sucroester WE 15, N.D. Gattefosse), les glycérides oléiques polyoxyéthylènes (Labrafil CS 2735; N.D. Gattefosse), les glycérides $C_8$-$C_{10}$ polyoxyéthylénés (Labrasol; N.D. Gattefosse) ou l'éther monoéthylique du diéthylèneglycol. Ces composés amphiphiles peuvent servir également de vecteurs de passage transdermique.

Ces émulsifiants non ioniques se différencient essentiellement par leur balance hydrophile-lipophile (HLB) et par leur température de fusion.

La cinétique de relargage du principe actif peut être déterminée en mesurant en fonction du temps la quantité cumulée de produit libéré dans un volume de liquide, à partir d'un film collé sur un support immergé dans le liquide selon la méthode de S. Borodkin et E.F. Tucker, J. Pharm. Sci., *64* (8) 1289—94 (1975). Le produit actif diffuse à travers la matrice avec une vitesse qui dépend essentiellement de la nature du polymère et de l'additif utilisé, mais qui est aussi fonction d'autres mécanismes tels que la dissolution du produit, lorsqu'il est cristallisé, ou le gonflement de la matrice.

D'après la loi de Fick, la quantité cumulée de produit libéré en fonction du temps peut être exprimée de la façon suivante:

$$Q = \sqrt{D(2A-Cs)Cp.t} = kr.\sqrt{t}$$

Dans cette formule,
Q représente la quantité cumulée de produit relargué par unité de surface (mg/cm2)
A représente la quantité de produit dans la matrice polymérique par unité de volume (mg/cm3)
Cs et Cp représentent la solubilité du produit dans le liquide et la matrice
et D représente le coefficient de diffusion du produit dans la matrice.

La représentation de cette fonction se traduit par une droite dont la pente (kr) est proportionnelle à la vitesse initiale de relargage du produit actif.

L'étude de l'influence des différents paramètres sur la valeur de kr permet de déterminer la composition de la forme transdermale adaptée à l'effet recherché.

A titre indicatif, l'influence des différents paramètres peut être étudiée de la manière suivant lorsque l'on utilise comme principe actif le dinitrate d'isosorbide et comme copolymère un copolymère polyuréthane-POEG-POPG et divers émulsifiants.

1° Influence de l'hydrophilie du copolymère

On prépare des films constitués de copolymères polyuréthane-POEG-POPG dans lesquels les rapports POEG/POPG sont 75/25, 50/50 et 25/75 contenant 10% en poids d'un émulsifiant non ionique (Plurol stéarique) et 10% en poids de dinitrate d'isosorbide et on mesure la quantité cumulée de produit actif libéré en fonction du temps.

Les résultats sont rassemblés dans le tableau I.

3

TABLEAU I

| Polyuréthane - POEG-POPG<br>$R = \dfrac{POEG}{POPG}$ | kr<br>mg/cm2.h$^{1/2}$ | temps de latence<br>$t_l$<br>(heure) |
|---|---|---|
| 75/25 | 1,06 | 0 |
| 50/50 | 0,6 | 0,3 |
| 25/75 | 0,5 | 0,77 |

Ces résultats montrent que la vitesse de libération est plus élevée pour un polymère hydrophile (POEG/POPG = 75/25) que pour un polymère hydrophobe (POEG/POPG = 25/75).

Par ailleurs d'autres mesures montrent que le polymère le plus hydrophile reprend jusqu'à 68% de son poids en eau tandis que le plus hydrophobe n'en reprend que 20% environ.

Dans tous les cas, la reprise en eau s'accompagne de la cristallisation du dinitrate d'isosorbide dans la matrice polymérique.

2° Influence de la nature de l'émulsifiant non ionique

On prépare des films constitués du copolymère polyuréthane POEG-POPG dans lequel le rapport POEG/POPG est égal à 75/25, des émulsifiants non ioniques et 10 à 20% de dinitrate d'isosorbide. On mesure la quantité cumulée de produit actif libéré en fonction du temps.

Les résultats sont rassemblés dans le tableau II.

TABLEAU II

| Emulsifiant non ionique | % d'émulsifiant en poids | dinitrate d'iso sorbide (%) | épaisseur du film (mm) | kr | temps de latence (h) |
|---|---|---|---|---|---|
| Sucro-ester WE 15 (solide) | 10 | 20 | 0,12 | 0,27 | 0 |
| | 20 | 10 | 0,21 | 0,83 | 0,13 |
| | 10 | 10 | 0,17 | 0,63 | 0 |
| Plurol stéarique (cire) | 10 | 10 | 0,11 | 0,92 | 0 |
| | 10 | 10 | 0,12 | 1,06-1,23 | 0 |
| Labrafil CS 2735 (liquide huileux) | 10 | 10 | | 0,95-1,1 | 0,18 |
| | 20 | 10 | 0,1 | 0,72 | 0,13 |
| Plurol isostéari- que (liquide) | 20 | 20 | 0,16 | | 0 |
| | 20 | 10 | 0,17 | | 0 |

De ces résultats il ressort que la vitesse d'élution du dinitrate d'isosorbide est influencée par le caractère lipophile et l'état physique (solide ou liquide) de l'émulsifiant utilisé.

Ainsi le Sucro-ester WE 15, bien que très hydrophile (balance hydrophile-lipophile = 15), ralentit la vitesse d'élution du dinitrate d'isosorbide. Avec le Labrafil CS 2735 (balance hydrophile-lipophile = 3—4), la vitesse d'élution est plus faible qu'avec le Plurol-stéarique (balance hydrophile-lipophile = 9—10), avec un temps de latence de 0,13 à 0,18 heure. Le temps de latence important est lié à la difficulté de gonflement du polymère.

L'addition de Plurol-isostéarique comme agent émulsifiant conduit à une augmentation de l'hydrophilie du polymère qui se traduit par un gonflement du polymère.

4

3° Influence de la concentration en dinitrate d'isosorbide

On prépare trois films à base de copolymère polyuréthane-POEG-POPG contenant 10% de Plurol stéarique comme émulsifiant et respectivement 10, 20 et 30% de dinitrate d'isosorbide. On mesure la quantité cumulée de produit actif libéré en fonction du temps.

Les résultats sont rassemblés dans le tableau III.

TABLEAU III

| Film | Dinitrate d'isosorbide % | Epaisseur du film (mm) | kr mg/cm2. h$^{-1/2}$ |
|------|------|------|------|
| N° 1 | 10 | 0,23 | 0,85 |
| N° 2 | 20 | 0,20 | 1,15 – 1,3 |
| N° 3 | 30 | 0,20 | 1,1 |

De ces résultats il ressort que le dinitrate d'isosorbide est relargué pratiquement avec une même vitesse initiale, indépendamment de la quantité initiale de produit actif.

Le film de copolymère contenant 30% de dinitrate d'isosorbide présente des difficultés de stockage. En effet, une concentration trop élevée en dinitrate d'isosorbide entraîne une cristallisation du produit actif au contact de l'humidité de l'air.

Selon la présente invention, les nouvelles formes transdermales peuvent être obtenues en ajoutant à un mélange du copolymère élastomère et d'un solvant organique, tel que le diméthylformamide, la méthyléthylcétone, le dioxanne, la méthylisobutylcétone, un mélange du principe actif et de l'émulsifiant non ionique en proportion déterminée. Le mélange est alors coulé, selon une technique connue, sur un support approprié, tel qu'une plaque de verre ou un support à adhérence contrôlée tel que du papier siliconé, après avoir éliminé, par évaporation sous pression réduite, une partie du solvant. Le film obtenu est alors séché sous pression réduite de façon à éliminer la totalité du solvant, en particulier dans le cas d'un solvant à point d'ébullition élevé.

Le mélange du copolymère et d'un solvant organique est préparé en agitant le copolymère élastomère et éventuellement le copolymère hydrophobe dans un solvant organique, tel que le diméthylformamide, à une température supérieure à 50°C de façon à obtenir un mélange homogène qui se présente en général sous forme d'un collodion. Généralement le mélange obtenu contient 5 à 30% en poids du copolymère ou du mélange de copolymères.

Le mélange du principe actif et de l'émulsifiant non ionique est préparé à partir d'une solution du principe actif dans un solvant organique convenable tel que l'alcool ou l'éther éthylique à laquelle est ajouté l'émulsifiant non ionique. Le mélange du principe actif et d'émulsifiant non ionique dans la proportion désirée est obtenu après évaporation du solvant éventuellement sous pression réduite.

Par exemple, avec le dinitrate d'isosorbide, il peut être avantageux de préparer des films à base de polyuréthane contenant 5 à 20% de dinitrate d'isosorbide, 5 à 30% d'émulsifiant non ionique et éventuellement 1 à 30% de l'additif hydrophobe, en poids par rapport au polymère mis en oeuvre.

Pour la mise en oeuvre des formes transdermales selon l'invention, il est nécessaire que les films ainsi préparés soient en contact aussi parfait que possible avec la peau. A cet effet, un adhésif pouvant avoir des propriétés des membranes homogènes, tel qu'un adhésif silicone, peut être appliqué à la surface du film par réticulation en présence d'un catalyseur convenable.

D'autres adhésifs peuvent être utilisés tels que par exemple ceux qui sont décrits dans le brevet américain US 3 797 494. Eventuellement, l'adhésif peut contenir du principe actif. Les adhésifs peuvent être appliqués para dépôt ou par transfert.

Par ailleurs, les films contenant le principe actif peuvent être recouverts sur les faces non en contact avec la peau de matériaux imperméables au relargage du principe actif qui peuvent, en outre, servir au maintien du dispositif sur la peau.

A titre indicatif, l'efficacité des formers transdermales selon la présente invention dans lesquelles le principe actif est le dinitrate d'isosorbide peut être mise en évidence chez l'animal et plus particulièrement chez le porc.

6 porcs de poids homogène sont répartis en 3 groupes de 2 animaux. Dans chaque groupe, un animal reçoit une dose déterminée et l'autre une dose double.

Le groupe A reçoit une crème de référence à 10% de dinitrate d'isosorbide.

Le groupe B reçoit un film à base de copolymère polyuréthane-POEG-POPG (75/25) et d'émulsifiant Plurol stéarique (10%) et le groupe C reçoit un film à base de copolymère polyuréthane-POEG-POPG (75/25) et d'émulsifiant Labrafil CS 2735 (10%).

Un animal d'un groupe reçoit 100 mg de dinitrate d'isosorbide et l'autre 200 mg de dinitrate d'isosorbide.

Pour chaque groupe d'animaux un prélèvement de sang de 5 à 10 cm3 est effectué avant l'administration de la dose puis à intervalles réguliers jusqu'à 8 heures aprés l'administration.

Le sang recueilli sur héparinate de lithium est placé à +4°C avant d'être centrifugé à 4500 tours/minute pendant 10 minutes.

le plasma est ensuite conservé à −20°C jusqu'au moment du dosage du dinitrate d'isosorbide par une méthode qui fait appel à la chromatographie en phase gazeuse sur colonne capillaire avec détection par capture d'électrons selon une technique dérivée de celle de H. LAUFEN et coll., J. Chromatog., *146*, 457 (1978).

Les films transdermiques sont évalués par rapport à la crème de référence sur:

— l'évolution des concentrations plasmatiques de dinitrate d'isosorbide au cours du temps

— les aires sous les courbes AUC donnant les concentrations de dinitrate d'isosorbide en fonction du temps.

Les résultats sont rassemblés dans le tableau IV.

TABLEAU IV

Concentrations plasmatiques de dinitrate d'isosorbide (DNIS) (ng/ml) obtenues
à l'aide des 3 modes d'administration transcutanée étudiés.
Quantités de DNIS résorbé (exprimées par l'aire sous les courbes AUC) à partir
des 3 modes d'administration transcutanée étudiés.

| Référence traitements | Traitement A crème | | Traitement B (émulsifiant Plurol) | | Traitement C (émulsifiant Labrafil) | |
|---|---|---|---|---|---|---|
| Doses de DNIS absorbées | 100 mg | 200 mg | 13,4 | 47,9 | 28 | 83 |
| "Débit" de DNIS ($\mu$g/mn) | 208,3 | 416,7 | 27,9 | 99,8 | 58,3 | 172,9 |
| Porc n° (sexe ; poids) | 2 F – 27,7 kg | 3 F – 28,4 kg | 4 F – 27,7 kg | 5 M – 28,5 kg | 6 M – 24,4 kg | 7 M – 25,3 kg |
| Temps de prélèvements | Concentrations plasmatiques de dinitrate d'isosorbide (ng/ml) | | | | | |
| 0 | 0 | 0 | 0 | 0 | 0 | 0 |
| 1 h | 3,60 | 42,65 | 0 | 0,65 | 0 | 0 |
| 2 h | 7,10 | 7,40 | 1,20 | 0,40 | 1,0 | 1,85 |
| 4 h | 5,60 | 8,95 | 17,30 | 5,55 | 2,85 | 1,30 |
| 6 h | 6,95 | 10,25 | 2,25 | 2,05 | 2,75 | 1,25 |
| 8 h | 3,15 | 10,45 | 1,60 | 2,10 | 1,75 | 1,90 |
| $AUC_{0 \rightarrow 8 h}$ (h.ng.ml$^{-1}$) | 42,50 | 102,60 | 42,50 | 18,55 | 14,45 | 9,78 |
| $AUC_{0 \rightarrow 8 h}$ normée à la dose (n.ng.ml$^{-1}$.mg$^{-1}$) | 0,425 | 0,513 | 3,172 | 0,387 | 0,516 | 0,118 |
| $AUC_{0 \rightarrow 8 h}$ normée * dose et poids | 11,77 | 14,57 | 87,86 | 11,03 | 12,59 | 2,98 |

* calculée selon la relation $= \dfrac{AUC_{0 \rightarrow 8h}}{Dose/poids} = \dfrac{AUC_{0 \rightarrow 8 h} \times poids}{Dose}$

Les résultats montrent en particulier que les films et la crème essayés s'opposent, en premier lieu, par la quantité de dinitrate d'isosorbide absorbée. En effet, si la résorption en 8 heurs est totale pour la crème elle n'est que de 15 à 25% pour les films.

L'absorption est plus importante à partir des films contenant du Labrafil. Cette différence sur les doses absorbées se retrouve aussi sur les concentrations plasmatiques de dinitrate d'isosorbide. Par ailleurs ces concentrations atteignent pour tous les films essayés des niveaux qui se sont toujours révélés efficaces en thérapeutique humaine.

L'examen des quantités résorbées dans le plasma indique que la résorption à partir des films est d'autant plus efficace que la dose théorique est plus faible. En outre elle est plus importante avec l'émulsifiant Plurol stéarique qu'avec l'émulsifiant Labrafil.

En résumé les films étudiés produisent in vivo pendant au moins 8 heures, des concentrations plasmatiques circulantes de dinitrate d'isosorbide qui atteignent des niveaux qui se sont révélés efficaces en thérapeutique humaine et la libération du dinitrate d'isosorbide à partir du support se fait de manière prolongée.

Les exemples suivants, donnés à titre non limitatif, montrent comment l'invention peut être mise en pratique.

EXEMPLE 1

A partir d'un solution éthanolique de dinitrate d'isosorbide à 20 g/l (30 cm3), on prépare un mélange avec 0,6 g de Plurol stéarique. Après évaporation du solvant, on obtient un mélange de 0,6 g de dinitrate d'isosorbide et de 0,6 g d'émulsifiant non ionique.

On prépare un collodion en agitant 4,8 g de copolymère polyuréthane-POEG-POPG (POEG-POPG = 75/25) dans 48 cm3 de diméthylformamide à une température de 60°C pendant 5 heures.

On ajoute 1,2 g du mélange de dinitrate d'isosorbide et de l'émulsifiant non ionique dans 53 cm3 du collodion préparé précédemment. Après évaporation partielle du diméthylformamide sous pression réduite, le collodion est coulé sur une plaque de verre en utilisant une couleuse à main réglable.

Le diméthylformamide est évaporé sous pression réduite (200 mm de mercure; 26,6 kPa) pendant 12 heures à 60°C.

On obtient ainsi un film d'aspect élatomérique qui est conditionné pour l'emploi après découpage en doses unitaires.

Le polymère polyuréthane/polyoxyéthylèneglycol-polyoxypropylèneglycol peut être préparé de la manière suivante:

On prépare le polyoxyéthylèneglycol fonctionnalisé en α, ω par un diisocyanate en faisant réagir à 80°C pendant 1,5 heure le diphénylméthanediisocyanate (MDI) sur le polyoxyéthylèneglycol α.ω-dihydroxylé (PM = 1500) préalablement déshydraté sous pression réduite (0,2 mm de mercure; 0,026 kPa) à 60°C en présence de malonitrile comme inhibiteur de réactions secondaires.

On prépare le polyoxypropylèneglycol fonctionnalisé en α,ω par un diisocyanate en faisant réagir pendant 40 minutes à 80°C le diphénylméthanediisocyanate (MDI) sur le polyoxypropylèneglycol α,ω-dihydroxylé (PM = 2000) en utilisant comme catalyseur du "Stavinor" et du malonitrile comme inhibiteur de réactions secondaires.

Les deux masses réactionnelles sont ensuite diluées à 20% dans le diméthylformamide pour stabiliser la réaction des groupements isocyanates sur les groupements hydroxyles.

Les deux solutions polymériques sont mélangées dans des proportions convenables et les polymères sont couplés au moyen de propane-1,2 diamine de telle sorte que le rapport amine/isocyanate soit égal à 0,95, ce rapport étant directement fonction de la masse moléculaire du polymère obtenu (71 000 daltons).

Le collodion à 20% dans le diméthylformamide est versé dans l'eau à 1% de chlorure de sodium. Le polymère est récupéré par centrifugation puis lavé 2 fois à l'eau distillée et enfin séché dans une étuve à 50°C sous pression réduite (200 mm de mercure; 26,6 kPa).

Exemple 2

On dissout 4,8 g de polymère commercialisé sous le nom de Pellethane 2102—75 dans 46 g de diméthylformamide.

A partir d'une solution éthanolique de dinitrate d'isosorbide à 20 g/l (30 cm3), on prépare un mélange avec 0,6 g de Plurol stéarique. L'alcool est évaporé sous pression réduite (100 nm de mercure; 13,3 kPa) à 50°C.

L'huile résiduelle est ajoutée au collodion de polymère préparé précédemment. Après homogénéisation, on obtient un collodion légèrement trouble dont la composition est la suivante:

| | |
|---|---|
| diméthylformamide | 88,5% |
| polymère Pellethane 2102—75 | 9,2% |
| dinitrate d'isosorbide | 1,15% |
| Plurol isostéarique | 1,15% |

8

Le collodion est coulé sur une plaque sous forme d'un film dont l'épaisseur initiale est de 0,6 mm. Le diméthylformamide est évaporé sous pression réduite (100 mm de mercure; 13,3 kPa) pendant 12 heures à 60°C.

On obtient un film légèrement trouble contenant 10% de dinitrate d'isosorbide dans lequel on découpe des disques de 45 mm de diamètre.

En atmosphère d'eau saturée, la reprise d'humidité est voisine de 5%.

La cinétique de relargage est effectuée en milieu aqueux et le dinitrate d'isosorbide libéré est dosé par HPLC. Les résultats sont rassemblés dans le tableau suivant:

| Temps (heures) | 1 | 4 | 24 | 48 | 72 |
|---|---|---|---|---|---|
| % de dinitrate d'isosorbide relargué | 8,7 | 2,9 | 51 | 55,4 | 65,6 |

Exemple 3

On opère de la même manière que dans l'exemple 2 mais en utilisant la méthyléthylcétone comme solvant, un polymère commercialisé sous le nom Estane et le Transcutol comme émulsifiant. On obtient un colloidion légérement trouble dont le composition est la suivante:

| | |
|---|---|
| méthyléthylcétone | 67% |
| polymère Estane | 23,1% |
| dinitrate d'isosorbide | 3,3% |
| éther monoéthylique du diéthylèneglycol | 6,6% |

Le collodion est coulé sur une plaque sous forme d'un film dont l'épaisseur initiale est de 1,25 mm. La méthyléthylcétone est évaporée sous pression réduite (100 mm de mercure; 13,3 kPa) pendant 12 heures à 60°C.

On découpe des disques de 45 mm de diamètre contenant 10% de dinitrate d'isosorbide qui sont hydratés en atmosphère saturée.

Les résultats de la cinétique de relargage en milieu aqueux sont rassemblés dans le tableau suivant:

| Temps (heures) | 1 | 4 | 24 | 48 | 72 |
|---|---|---|---|---|---|
| % de dinitrate d'isosorbide relargué | 11,7 | 17,7 | 68 | 83,4 | 92,5 |

Exemple 4

Dans 40 g de collodion composé de 28 g de méthyléthylcétone, 9,6 d'Estane et de 2,4 de Labrasol, on ajoute une solution constituée de 9 g de méthyléthylcétone, 3,2 g de kétoprofène et 0,8 g de Labrasol.

Après homogénéisation pendant 10 minutes à 45°C sous atmosphère d'azote, on obtient un collodion limpide dont la composition est la suivante:

| | |
|---|---|
| méthyléthylcétone | 69,8% |
| Estane | 18,1% |
| kétoprofène | 6,05% |
| Labrasol | 6,05% |

Le collodion est coulé sur une plaque sous forme d'un film dont l'épaisseur initiale est de 1 mm. Après évaporation du solvant dans les conditons habituelles, on obtient un film homogène et transparent contenant 20% de kétoprofène qui est transféré sur du papier siliconé.

On découpe des disques de 45 mm de diamètre. En atmosphère d'eau saturée, la reprise d'humidité est voisine de 69%.

Les résultats de la cinétique de relargage du kétoprofène dans l'eau pure sont rassemblés dans la tableau suivant: (le dosage du kétoprofène est effectué par dosage en spectrographie ultraviolette à 255 nm)

| Temps (heures) | | 0,5 | 1 | 4 | 6 | 22 | 30 | 93 | 105 |
|---|---|---|---|---|---|---|---|---|---|
| % de kétoprofène relargué | 1er essai | 1,9 | 3,6 | 7,5 | 11,2 | 23 | 24,6 | 30,5 | 32,6 |
| | 2e essai | 1,8 | 3,5 | 7,2 | 9,8 | 18,5 | 21,6 | 26 | 29,4 |
| | 3e essai | 2,3 | 3,5 | 9,1 | 11,6 | 18,8 | 21,4 | 29,6 | 38,7 |

## Exemple 5

On prépare un collodion dont la composition est la suivante:

| | |
|---|---|
| méthyléthylcétone | 64,4% |
| éthanol | 14,4% |
| eau | 3% |
| Estane | 11% |
| chlorhydrate d'acébutolol | 3,6% |
| Labrasol | 3,6% |

(Le chlorhydrate d'acébutolol étant soluble dans l'eau, il est nécessaire d'ajouter de l'éthanol et de l'eau pour obtenir un collodion homogène et transparent).

La préparation du film et son séchage sont effectués dans les conditions décrites dans l'exemple 2. On obtient un film translucide parsemé de cristaux qui contient 20% de chlorhydrate d'acébutolol.

Les résultats de la cinétique de relargage du chlorhydrate d'acébutolol en milieu aqueux sont rassemblés dans le tableau suivant: (le dosage du chlorhydrate d'acébutolol est effectué par dosage en spectrographie ultra-violette à 235 nm)

| Temps (heures) | | 0,25 | 0,50 | 1 | 2 |
|---|---|---|---|---|---|
| % de chlorhydrate d'acébutolol relargué | 1er essai | 30,8 | 41,1 | 58,3 | 89,3 |
| | 2e essai | 28,6 | 33,6 | 52,3 | 88,7 |
| | 3e essai | 23,8 | 30,1 | 39 | 94,4 |

## Exemple 6

On prépare un collodion ayant la composition suivante:

| | |
|---|---|
| méthyléthylcétone | 58% |
| éthanol à 95° | 12% |
| Estane | 18% |
| tartrate de nicergoline | 6% |
| Labrasol | 6% |

Le collodion est coulé sur une plaque sous forme d'un film dont l'épaisseur initiale est de 0,5 mm.

Après séchage dans les conditions habituelles, on obtient un film homogène quasiment transparent contenant 20% de tartrate de nicergoline.

On découpe des disques de 45 mm de diamètre.

Les résultats de la cinétique de relargage du chlorhydrate de nicergoline en milieu aqueous sont rassemblés dans le tableau suivant: (le tartrate de nicergoline est dosé par spectrographie ultra-violette à 277 nm)

**0 123 585**

| Temps (heures) | | 0,25 | 0,75 | 1,25 | 3,25 |
|---|---|---|---|---|---|
| % de tartrate de nicergoline relargué | 1er essai | 1,32 | 3,5 | 14,1 | 52,8 |
| | 2e essai | 0,75 | 5,9 | 16,7 | 43,1 |

Exemple 7

On prépare un collodion homogène ayant la composition suivante:

| | |
|---|---|
| méthyléthylcétone | 37,7% |
| chloroforme | 37,7% |
| polymère | 18,1% |
| métopimazine | 2% |
| Labrasol | 4,5% |

Le collodion est coulé sur une plaque sous forme d'un film dont l'épaisseur initiale est de 1 mm. Après séchage dans les conditions habituelles, on obtient un film légèrement jaune, homogène et translucide contenant 8,1% de métopimazine.

On découpe des disques de 45 mm de diamètre.

Les résultats de la cinétique de relargage de la métopimazine en milieu aueous sont rassemblés dans la tableau suivant:

(la métopimazine est dosée par spectrographie ultra-violette à 263 nm)

| Temps (heures) | | 0,5 | 1 | 4,5 | 6 | 21 | 45 | 142 | 190 |
|---|---|---|---|---|---|---|---|---|---|
| % de métopimazine relarguée | 1er essai | 0,36 | 1,3 | 4,7 | 5,1 | 12,1 | 27,3 | 60,2 | 75,3 |
| | 2e essai | 0,50 | 3 | 4,7 | 5 | 15,7 | 23,5 | 69,2 | 78,3 |

Exemple 8

Dans 437 cm3 de méthyléthylcétone on dissout 30 g de dinitrate d'isosorbide et 30 g d'émulsifiant non ionique (Labrasol). Dans le solution agitée vigoureusement on introduite 90 g de polyuréthane (Estane 5702) en granulés. L'agitation est maintenue pendant 1 heure. Le collodion ainsi obtenu est laissé au repos pendant 14 heures de façon à en éliminer les bulles.

Une épaisseur constante de collodion est étendue sur une bande de papier siliconé double face qui défile dans un tunnel de ventilation et dans un four chauffé à 70—80°C de façon à éliminer efficacement le solvant par évaporation. Le complexe film actif/papier siliconé est roulé sur lui-même pour en permettre le stockage.

Le film actif ainsi préparé est ensuite collé par tranfert sur une bande de support protecteur du film actif (polyéthylène, PVC, Terphane éventuellement aluminisé et pigmenté).

Sur la partie active du film recouvert d'un support protecteur, on déposé par transfert un film d'adhésif préalablement préparé par enduction sur papier antiadhérent. On obtient ainsi un complexe comportant une couche de support protecteur, le film actif, le film adhésif et le papier antiadhérent. Le film adhésif est constitué d'un matériau sensible à la pression tel que des copolymères d'esters d'acide polyacrylique ou polyméthacrylique, des alcoyléthers polyvinyliques, des polyuréthanes, des polyesters, des polyamides, des copolymères d'éthylène-acétate de vinyle, des copolymères blocs styrène-isoprène, des gommes polyisobutylènes ou des résines silicones.

Le complexe ainsi préparé est ensuite découpé à l'emportepièce à la forme et à la taille désirée. Les "patches" ainsi obtenus sont placés dans des sachets thermosoudés convenablement choisis pour un stockage prolongé.

Il peut être avantageux de découper le film actif avant de le transférer sur le support protecteur puis de découper un complexe (film adhésif) de surface plus grande que celle du film actif afin d'obtenir une masse adhésive à la périphérie du film actif.

Le film adhésif peut être chargé en principe actif (1 à 10%) afin d'éviter un temps de latence de libération du principe actif.

11

# 0 123 585

**Revendications pour les Etats contractants: BE CH DE FR GB IT LI LU NL SE**

1. Forme transdermale d'un médicament caractérisée en ce que le principe actif est dispersé sous forme cristallisée ou solubilisée dans un polymère thermoréversible non réticulé filmogène homogène insoluble dans l'eau, choisi parmi les copolymères polyuréthane-polyoxyéthylèneglycol-polyoxypropylèneglycol ou les polyuréthanes à base polyéther ou polyester de polytétraméthylèneglycol éventuellement avec un agent émulsifiant non ionique.

2. Forme transdermale selon la revendication 1 caractérisée en ce que l'émulsifiant non ionique est choisi dans le groupe constitué par le palmitostéarate de polyglycérol, l'isostéarate de polyglycérol, le monopalmitate de saccharose, le glycéride oléique polyoxyméthylène, éther monoéthylique du diéthylèneglycol et les glycérides $C_8$—$C_{10}$ polyoxyéthylénes.

3. Forme transdermale selon la revendication 1 caractérisé en ce qu'elle contient 10 à 20% de principe actif et 10 à 20% d'émulsifiant non ionique, en poids par rapport à la matrice élastomère mise en oeuvre.

4. Forme transdermale selon l'une des revendications 1 ou 3 caractérisée en ce que la composition est recouverte d'un adhésif.

5. Forme transdermale selon la revendication 1 caractérisée en ce que le principe actif est choisi parmi les antiangoreux, les antiémétiques, les anti-cholinergiques, les anti-inflammatoires non stéroïdiens, les anti-inflammatoires stéroïdiens, les anti-hypertenseurs, les béta-bloquants, les vasodilatateurs, les neuroleptiques, les tranquillisants, le bronchodilatateurs, les antispasmodiques et les hormones.

6. Forme transdermale selon la revendication 1 caractérisée en ce que le principe actif est choisi parmi le dinitrate d'isosorbide, le mononitrate d'isosorbide, la trinitrine, la métopimazine, le dimenhydrinate, la scopolamine, le kétoprofène, la prednisolone, la dexaméthazone, la clonidine, l'acébutolol, le nicergoline, les phénothiazines neuroleptiques, les benzodiazépines tranquillisantes, la suriclone, la suproclone, la phénylpropanolamine, le bromure de butylhyoscine et l'estradiol.

7. Procédé de préparation d'une forme transdermale selon la revendication 1 caractérisé en ce que l'on ajoute au copolymère élastomère éventuellement dans un solvant organique un mélange du principe actif et de l'émulsifiant non ionique en proportion déterminée, coule sur un support approprié le mélange obtenu éventuellement après évaporation partielle du solvant, puis sèche le film obtenu pour éliminer la totalité de solvant et le recouvre éventuellement d'un adhésif.

8. Forme transdermale selon l'une des revendication 1 ou 3 lorsqu'elle est conditionnée en dose unitaire pour son application en thérapeutique humaine.

**Revendications pour l'Etat contractant: AT**

1. Procédé de préparation d'une forme transdermale d'un médicament caractérisé en ce que l'on ajoute à un polymère thermoréversible non réticulé, filmogène, insoluble dans l'eau choisi parmi les copolymères polyuréthane-polyoxyéthylèneglycol-polyoxypropylèneglycol ou les polyuréthanes à base polyéther ou polyester de polytétraméthylèneglycol, éventuellement dans un solvant organique, un mélange du principe actif et d'un émulsifiant non ionique en proportion déterminée, coule sur un support approprié le mélange obtenu, éventuellement après évaporation partielle du solvant, puis sèche le film obtenu pour éliminer la totalité du solvant et la recouvre éventuellement d'un adhésif.

2. Procédé selon la revendication 1 caractérisé en ce que l'emulsifiant non ionique est choisi dans le groupe constitué par le palmitostéarate de polyglycérol, l'isostéarate de polyglycérol, le monopalmitate de saccharose, le glycéride oléique polyoxyméthylène, éther monoéthylique du diéthylèneglycol et les glycérides $C_8$—$C_{10}$ polyoxyéthylénés.

3. Procédé selon la revendication 1 caractérisé en ce que l'on utilise 10 à 20% de principe actif et 10 à 20% d'emulsifiant non ionique en poids par rapport à la matrice élastomère mise en oeuvre.

4. Procédé selon l'une des revendications 1 ou 3 caractérisé en ce que le principe actif est choisi parmi les antiangoreux, les antiémétiques, les anticholinergiques, les anti-inflammatoires non stéroidiens, les anti-inflammatoires stéroïdiens, les anti-hypertenseurs, les béta-bloquants, les vasodilateurs, les neuroleptiques, les tranquillisants, les bronchodilatateurs, les antispasmodiques et les hormones.

5. Procédé selon l'une des revendications 1 ou 3 caractérisé en ce que le principe actif est choisi parmi le dinitrate d'isosorbide, le mononitrate d'isosorbide, la trinitrine, le métopimazine, le dimenhydrinate, la scopolamine, le kétoprofène, la prednisolone, la dexaméthazone, le clonidine, l'acébutolol, la nicergoline, les phénothiazines neuroleptiques, les benzodiazépines tranquillisantes, la suriclone, la suproclone, la phénylpropanolamine, le bromure de butylhyoscine et l'estradiol.

**Patentansprüche für die Vertragsstaaten: BE CH DE FR GB IT LI LU NL SE**

1. Transdermale Arzneimittelform, dadurch gekennzeichnet, daß das aktive Prinzip bzw. der Wirkstoff in kristallisierter oder solubilisierter Form dispergiert ist in einem thermoreversiblen, nicht vernetzten, filmbildenden, homogenen, in Wasser unlöslichen Polymeren, ausgewählt unter den Copolymeren Polyurethan-polyoxyethylenglykol-polyoxypropylenglykol oder den Polyurethanen auf Basis von Polytetramethylenglykol-polyether oder -polyester, gegebenenfalls mit einem nicht-ionischen Emulgiermittel.

2. Transdermale Form gemäß Anspruch 1, dadurch gekennzeichnet, daß das nicht-ionische Emulgiermittel ausgewählt ist aus der Gruppe bestehend aus Polyglycerinpalmitostearat, Polyglycerinisostearat, Saccharosemonopalmitat, Polyoxyméthylenöleinglycerid, Monoethylether des Diethylenglykols und den polyoxyethylierten $C_8$—$C_{10}$-Glyceriden.

3. Transdermale Form gemäß Anspruch 1, dadurch gekennzeichnet, daß sie 10 bis 20% Wirkstoff und 10 bis 20% nicht-ionisches Emulgiermittel, bezogen auf das Gewicht der eingesetzten Elastomerenmatrix, enthält.

4. Transdermale Form gemäß einem der Ansprüche 1 oder 3, dadurch gekennzeichnet, daß die Zusammensetzung mit einem Haftmittel bedeckt ist.

5. Transdermale Form gemäß Anspruch 1, dadurch gekennzeichnet, daß der Wirkstoff ausgewählt ist unter den Mitteln gegen Angina pectoris, den Antiemetika, den Anticholinergika, den nicht-steroiden antiinflammatorischen Mitteln, den steroidantiinflammatorischen Mitteln, den Antihypertensiva, den Betablockern, den Vasodilatoren, den Neuroleptika, den Tranquilizern, den Bronchodilatoren, den Antispasmodika und den Hormonen.

6. Transdermale Form gemäß Anspruch 1, dadurch gekennzeichnet, daß der Wirkstoff ausgewählt ist unter Isosorbid-dinitrat, Isosorbid-mononitrat, Trinitrin, Metopimazin, Dimenhydrinat, Schopolamin, Ketoprofen, Prednisolon, Dexamethason, Clonidin, Acebutolol, Nicergolin, den neuroleptischen Phenothiazinen, den tranquillisierenden Benzodiazepinen, dem Suriclon, Suproclon, Phenylpropanolamin, Butylhyoscinbromid und Estradiol.

7. Verfahren zur Herstellung einer transdermalen Form gemäß Anspruch 1, dadurch gekennzeichnet, daß man dem elastomeren Copolymeren, gegebenenfalls in einem organischen Lösungsmittel, ein Gemisch des Wirkstoffs und des nicht-ionischen Emulgators in einem bestimmten Mengenverhältnis zusetzt, das erhaltene Gemisch, gegebenenfalls nach teilweiser Verdampfung des Lösungsmittels, auf einen geeigneten Träger gießt, dann den erhaltenen Film trocknet, un das gesamte Lösungsmittel zu entfernen und ihn gegebenenfalls mit einem Haftstoff bedeckt.

8. Transdermale Form gemäß einem der Ansprüche 1 oder 3, konditioniert in einer Einheitsdosis zur Anwendung in der Humantherapie.


**Patentansprüche für den Vertragsstaat: AT**

1. Verfahren zur Herstellung einer transdermalen (durch die Haut zuführebaren) Form eines Medikaments, dadurch gekennzeichnet, daß man einem wasserunlöslichen, filmbildenden, nicht vernetzten, thermoreversiblen Polymer, ausgewählt aus den Polyurethan/Polyoxyäthylenglykol/Polyoxypropylenglykol-Copolymeren oder den Polyurethanen auf Basis von Polytetramethylenglykolpolyäther oder -polyester, gegebenenfalls in einem organischen Lösungsmittel, eine Mischung des aktiven Bestandteils und eines nicht-ionischen Emulgiermittels in festgelegten Anteilen zusetzt, die erhaltene Mischung, gegebenenfalls nach Teilweisem Abdampfen des Lösungsmittels, auf einen geeignet Träger gießt und dann den erhaltenen film zur Entfernung des gesamten Lösungsmittels trocknet und ihn gegebenenfalls mit einem Klebstoff beschichtet.

2. Verfahren nach Anspruch 1, dadurch gekennzeichnet, daß das nicht-ionische Emulgiermittel ausgewählt ist aus der Gruppe, bestehend aus Polyglycerinpalmitostearat, Polyglycerinisosostearat, Saccharosemonopalmitat, Polyoxymethylenoleinglycerid, Diäthylenglykolmonoäthyläther und den Polyoxyäthylen-$C_8$—$C_{10}$-glyceriden.

3. Verfahren nach Anspruch 1, dadurch gekennzeichnet, daß man 10 bis 20% aktiven Bestandteil und 10 bis 20% nicht-ionisches Emulgiermittel, bezogen auf das Gewicht der eingesetzten Elastomer-Grundmasse, verwendet.

4. Verfahren nach einem der Ansprüche 1 oder 3, dadurch gekennzeichnet, daß der aktive Bestandteil ausgewählt ist aus den Mitteln gegen Angina pectoris, den Antiemetika, den Anticholinergika, den nicht steroidalen entzündungshemmern, den steroidalen Entzündungshemmern, den Blutdrucksenkenden Mitteln, den Betablockern, den Vasodilatatoren, den Neuroleptika, den Tranquillizern, den Bronchodilatatoren, den Spasmolytika und den Hormonen.

5. Verfahren nach einem der Ansprüche 1 oder 3, dadurch gekennzeichnet, daß der aktive Bestandteil ausgewählt ist aus Isosorbiddinitrat, Isosorbidmononitrat, Trinitrin, Metopimazin, Dimenhydrinat, Scopolamin, Ketoprofen, Prednisolon, Dexamethason, Clonidin, Acebutolol, Nicergolin, den neuroleptischen Phenothiazinen, den Benzodiazepin-Tranquillizern, Suriclon, Suproclon, Phenylpropanolamin, Butylhyoscinbromid und Östradiol.


**Claims for the Contracting States: BE CH DE FR GB IT LI LU NL SE**

1. A transdermal form of a medicament, in which the active principle is dispersed in crystalline or solubilized form in a non-crosslinked, film-forming homogeneous heat-reversible polymer which is insoluble in water, chosen from polyurethane/polyoxyethylene glycol/polyoxypropylene glycol copolymers, or polyurethanes based on a polyether or polyester of polytetramethylene glycol, optionally with a non-ionic emulsifying agent.

2. A transdermal form according to claim 1, in which the non-ionic emulsifier is chosen from the group comprising polyglycerol palmitostearate, polyglycerol isostearate, sucrose monopalmitate, polyoxymethylene oleyl glyceride, diethylene glycol monoethyl ether and polyoxyethyleneated $C_8$—$C_{10}$-glycerides.

3. A transdermal form according to claim 1, which contains 10 to 20% of active principle and 10 to 20% of nonionic emulsifier, the percentages being by weight, based on the elastomeric matrix used.

4. A transdermal form according to either of claims 1 and 3, in which the composition is covered by an adhesive.

5. A transdermal form according to claim 1, in which the active principle is chosen from antianginal agents, antiemetic agents, anticholinergic agents, non-steroid antiphlogistic agents, steroid antiphlogistic agents, antihypertensive agents, beta-blockers, vasodilators, neuroleptic agents, tranquillizers, bronchodilators, antispasmodic agents and hormones.

6. A transdermal form according to claim 1, in which the active principle is chosen from isosorbide dinitrate, isosorbide mononitrate, trinitrin, metopimazine, dimenhydrinate, scopolamine, ketoprofene, prednisolone, dexamethazone, clonidine, acebutolol, nicergoline, neuroleptic phenothiazines, tranquillizing benzodiazepines, suriclone, suproclone, phenylpropanolamine, butylhyoscine bromide and estradiol.

7. A process for the preparation of a transdermal form according to claim 1, which comprises adding to the elastomeric copolymer, optionally in an organic solvent, a mixture of the active principle and the non-ionic emulsifier in a defined proportion, pouring the mixture obtained onto a suitable support, optionally after partial evaporation of the solvent, then drying the film obtained to remove all the solvent and optionally coating it with an adhesive.

8. A transdermal form according to either of claims 1 and 3, packaged in a unit dose for use in human therapy.

**Claims for the Contracting State AT**

1. A process for the preparation of a transdermal form of a medicament, which comprises adding to a non-crosslinked, film-forming heat-reversible polymer which is insoluble in water, chosen from polyurethane/polyoxyethylene glycol/polyoxypropylene glycol copolymers, or polyurethanes based on a polyether or a polyester of polytetramethylene glycol, optionally in an organic solvent, a mixture of the active principle and a non-ionic emulsifier in a defined proportion, pouring the mixture obtained onto a suitable support, optionally after partial evaporation of the solvent, then drying the film obtained to remove all the solvent and optionally coating it with an adhesive.

2. A process according to claim 1, in which the nonionic emulsifier is chosen from the group comprising polyglycerol palmitostearate, polyglycerol isostearate, sucrose monopalmitate, polyoxymethylene oleyl glyceride, diethylene glycol monoethyl ether and polyoxyethyleneated $C_8$—$C_{10}$-glycerides.

3. A process according to claim 1, which comprises employing 10 to 20% of active principle and 10 to 20% of non-ionic emulsifier, the percentages being by weight, based on the elastomeric matrix used.

4. A process according to either of claims 1 and 3, in which the active principle is chosen from antianginal agents, antiemetic agents, anticholinergic agents, nonsteroid antiphlogistic agents, steroid antiphlogistic agents, antihypertensive agents, beta-blockers, vasodilators, neuroleptic agents, tranquillizers, bronchodilators, antispasmodic agents and hormones.

5. A process according to either of claims 1 and 3, in which the active principle is chosen from isosorbide dinitrate, isosorbide mononitrate, trinitrin, metopimazine, dimenhydrinate, scopolamine, ketoprofene, prednisolone, dexamethazone, clonidine, acebutolol, nicergoline, neuroleptic phenolthiazines, tranquillizing benzodiazepines, suriclone, suproclone, phenylpropanolamine, butyl-hyoscine bromide and estradiol.

14